# EUROPEAN PATENT SPECIFICATION

(11) **EP 1 718 320 B1**
(45) Date of publication and mention of the grant of the patent: **31.03.2010**
(21) Application number: 05702144.6
(22) Date of filing: 07.02.2005
(51) Int. Cl.: A61K 36/55, A61P 1/02, A61P 1/00

(54) **LINSEED EXTRACT FOR XEROSTOMIA TREATMENT**
LEINSAMENEXTRAKT ZUR BEHANDLUNG VON XEROSTOMIE
EXTRAIT DE GRAINES DE LIN UTILISE POUR TRAITER LA XEROSTOMIE

(30) Priority: 05.02.2004 GB 0402539
(43) Date of publication of application: 08.11.2006
(73) Proprietor: Sinclair Pharmaceuticals Limited, Godalming, Surrey GU7 2AB (GB)
(72) Inventor: ARNEBRANT, Thomas, S-223 59 Lund (SE); ELOFSSON, Ulla, S-172 32 Sundbyberg (SE); LARSSON, Kåre, S-237 34 Bjärred (SE)
(74) Representative: Jappy, John William Graham
(86) International application number: PCT/GB2005/000426
(87) International publication number: WO 2005/074957

(56) References cited:
- EP-A- 0 511 181
- WO-A-93/16707

## Description

### Field of Invention

The present invention is concerned with the treatment of xerostomia, in particular, with solid pharmaceutical preparations used in the treatment of xerostomia.

### Background of the Invention

A reduced salivary secretion may arise or develop with increasing age. Thus, a large number of elderly people have problems with a dry mouth. Some general diseases also give rise to a reduced secretion of saliva, so called hyposalivation. The most prominent one thereof is Sjoegren's syndrome. Furthermore, several generally used medicines, inter alia, anti-hypertensives, anti-ulcer agents and anti-psychotics, have side effects including hyposalivation. Reduced salivary gland function may also accompany or follow a variety of anti-cancer treatments including radiotherapy and chemotherapy. In other words dryness of the mouth, or xerostomia, is a common disease that affects a large number of the population transiently or permanently. The reduced secretion of saliva can cause a variety of subjective symptoms including discomfort of of the tongue, mouth, pharynx and upper esophagus, sensitivity to spicy food and beverages and loss of sleep. Some individuals also have their speech and swallowing affected.

Objectively dryness of the mouth often causes caries and periodontitis, which are difficult to treat since the reduced secretion of saliva results in a more pronounced retention of bacteria in the oral cavity and on the teeth. The resistance of the mucosa against colonization of bacteria is reduced and especially fungal infections are common in connection with individuals with xerostomia. Furthermore, people carrying a plate prosthesis often have great problems with the retention of the prosthesis as well as infection of the mucosa as consequences of the dryness of the mouth. Other objective signs may include halitosis and recurrent ulcers in the mouth and oropharynx. Natural saliva consists of highly specialised proteins, which are strongly surface-active. They thus form surface films at interfaces of solids and also at soft tissues such as the surfaces of the oral cavity. This film also functions as a lubricant.

US Patent No. 5,260,282 discloses a saliva substitute comprising water-soluble linseed polysaccharides. Said substitute is presented in the form of an aqueous solution. The substitute can be prepared by extracting the polysaccharides from the linseed by means of water or a water solution containing inorganic salts.

EP-A-0511181 discloses a saliva substitute comprising water-soluble linseed polysaccharides. The saliva substitute is preferably an aqueous solution having a viscosity within the range 1-30mPa.S.

WO-A-93/16707 discloses compositions comprising linseed mucilage and purified forms thereof having therapeutic and cosmetic utility for topical application to the skin and/or mucous membranes of the body. The linseed mucilage compositions have mucoadherent properties and may be used as a artificial mucus or lubricant.

The types of saliva substitutes, such as those described above, consists of polysaccharides, often chemically modified natural products such as cellulose derivatives, for example carboxymethyl cellulose. They provide viscosity, but only very limited surface activity. The extraction procedure used to prepare linseed extract gives beside polysaccharides a considerable amount of proteins. These proteins are also quite surface active as seen by adsorption measurements. Linseed extract can emulsify oil and this is also a desired property of a saliva substitute, since oils in the food must be dispersed into water phase.

### Summary of Invention

In accordance with the present invention, there is provided the use of a water-soluble or water-dispersible linseed extract **characterised in that** the extract has an absorption of at least 1.2mg/m² wherein the adsorption is measured by contacting an aqueous solution or dispersion of the extract with silica substrate, rinsing the silica substrate and then measuring the adsorption by ellipsometry, in the manufacture of a solid medicament for the treatment of xerostomia by direct administration of the medicament in a solid form.

The salivary glands of the mouth normally produce around 1-1.5 litres of saliva per 24 hours, and it must be considered unrealistic to utilize a saliva substitute that has to be taken in such a volume per 24 hours. Thus, the present invention provides an alternative to liquid saliva substitutes, while providing a number of advantageous physical properties discussed below.

Linseed contains polysaccharides and proteins exhibiting physical properties, which are similar to those of mixed saliva. For patients with reduced saliva production, who comprise the majority of patients with xerostomia, a solid dosage form, for example a tablet, does not at first sight make sense. However, a dry formulation is more convenient because it can be used discreetly, is easily carried and can have slow release characteristics and greater control on the duration of action. It may further provide benefits of higher stability, easier handling, easier transportation and lower manufacturing and packaging costs.

It has now been found that the process of drying the linseed extract has a surprising effect on the physical properties of the linseed extract. A surprising increase in adsorption to surfaces, especially to tissue, in particular mucosal tissue, is observed. Without wishing to be bound by theory, it is postulated that these changes in physical properties are attributable to changes in the structure of the proteins associated with the linseed extract. Such changes have particular advantages in treatment of xerostomia as an increased adsorption leads to longer residence times on tissue and smaller dosage requirements. Additionally, a resultant solution has improved film-forming properties and gives a much improved mouth feel. This latter advantage is attributed to the solution having a similar viscosity and lubricity to natural saliva.

As discussed above, it is known that the mixture of proteins and polysaccharides present in linseed extract possess a very unusual combination of rheological and surface-chemical properties, which make them extremely suitable for the application described in the present invention. Prior art teachings suggest that the linseed extract should be applied to the patient in the form of an aqueous composition, effectively a saliva substitute. The present invention teaches quite the contrary. The present invention teaches the administration of a solid composition to the patient. This is contrary to what one would expect when treating patients suffering from xerostomia.

Such solid preparations provide a convenient metered dose, a discrete packaging and form of administration to the patient and substantially less packaging then an aqueous product.

A composition of the present invention in solid form is directly administrable to a patient. That is to say that a solid formulation is used in the treatment, rather than have to be made up into a solution. A composition of the present invention in solid form find particular utility in patients suffering from mild to moderate xerostomia.

The linseed extract is of the type that is obtainable by a simple extraction in water of said polysaccharides and proteins directly from linseed as described in patent no. US 5,260,282, which is incorporated herein by reference. Of course, any extraction method may be employed, for example extraction with an organic solvent alone or with water, a supercritical fluid or a mixture of the above. Where a mixture of an organic solvent and water is used, preferably a protic solvent such as ethanol is utilised.

One advantage of using a supercritical fluid to extract the polysaccharide and protein fraction from the linseed is that it is extremely easy to remove the solvent from the extract, thus reducing the process steps in order to arrive at a solid product.

The linseed extract used in the present invention may be obtained by simple dissolution or extraction from linseed in water at ambient temperature, however elevated temperatures and/or pressures may be utilised in the extraction.

In a particularly preferred embodiment, the extract of the present invention is produced by spray drying or freeze drying solutions, preferably aqueous solutions comprising the linseed extract. Conventional spray drying and freeze drying techniques may be employed. Spray drying and freeze drying techniques have been shown to provide advantageous processing features which have the effect of altering the structure of the proteins associated with the linseed extract. Spray drying is especially preferred as this leads to the most marked change in the physical properties of the linseed extract, for example the adsorption characteristics.

Preferably, spray drying takes place at a temperature of greater than 110°C, preferably greater than 135°C, more preferably greater than 150°C, most preferably greater than 170°C, for example, about 180°C.

The adsorption of an aqueous solution, as measured using ellipsometry on a silica substrate, formed by dissolution of the extract or composition according to the invention preferably has an adsorption in the range of from 1.3 to 5 mg/m², more preferably from 1.4 to 4 mg/m², more preferably 1.5-3 mg/m². Where the extract according to the invention has been prepared according to the invention by spray drying, improved adsorption characteristics are obtained and the adsorption is preferably from 1.75 to 2.5 mg/m², more preferably about 2 mg/m².

In the adsorption measurement method, there is a contact time between when the aqueous solution or dispersion of the extract contacts the silica substrate and when the silica substrate is rinsed wherein the contact time is from 100 to 3000 seconds, preferably from 500 to 2500 seconds, more preferably from 1000 to 2000 seconds.

Preferably the composition of the present invention is substantially free of water. The composition has preferably less than 10% water by weight of composition, more preferably less than 5% water by weight, more preferably less than 2% water by weight, most preferably less than 1% water by weight.

A number of additives may be advantageously included in the composition of the present invention.

In a particularly preferred embodiment a sialogogue is present in the composition according to the invention. Preferred sialogogues include pharmaceutically acceptable organic acids such as citric acid, malic acid, ascorbic acid, fumaric acid and the like. Malic acid is a particularly preferred sialogogue.

In a particularly preferred embodiment, a lubricious polymer is included in the composition according to the invention. This aids in the disintegration of the composition and in dispersing the composition around the oral cavity. Preferred polymers are selected from alkylene oxide polymers and interpolymers and derivatives thereof where the terminal hydroxyl groups have been modified by esterification, etherification, etc. These are exemplified by the compounds prepared through polymerization of ethylene oxide or propylene oxide and the alkyl and aryl ethers of these polyoxyalkylene polymers. Casein fractions can also provide this function.

A suitable route of administration for the extract and/or the composition according to the invention is oral administration.

An extract of the present invention may be administered to a patient either alone or mixed with suitable carriers or excipient(s) at a dose suitable to treat or ameliorate xerostomia. Such a composition may also contain diluents, fillers, salts, buffers, stabilizers, solubilizers, binders, disintegrators, thickeners and other materials known in the art. The term "pharmaceutically acceptable" means a non-toxic material that does not interfere with the effectiveness of the biological activity of the active ingredient(s).

A pharmaceutical composition according to the present invention may be formulated in a conventional manner using one or more pharmaceutically acceptable carriers comprising an excipient and auxiliary which facilitates processing of the active component(s) of the composition into a preparation which can be used pharmaceutically. The pharmaceutical composition according to the invention may be manufactured in a manner that is itself known, e.g., by means of a conventional mixing, dissolving, granulating, dragee-making, levigating, emulsifying, encapsulating and/or entrapping process. Most preferably, spray drying or freeze drying are used to prepare a pharmaceutical preparation which may then undergo further processing to produce, for example, a tablet or the like.

When a therapeutically effective amount of the composition of the present invention is administered orally, the composition will preferably be in the form of a tablet, capsule or powder. For oral administration, the composition according to the invention can be formulated readily by combining the active component(s) with a pharmaceutically acceptable carrier, as is well known in the art. Such a carrier enables the extract of the invention to be formulated as a tablet, pill, dragee, capsule and the like, for oral administration to a patient to be treated.

A preferred excipient is, in particular, a filler such as a sugar, including lactose, sucrose, mannitol, xylitol, galactitol, isomaltose or sorbitol and mixtures thereof; a cellulose preparation such as, for example, maize starch, wheat starch, rice starch, potato starch, gelatin, gum tragacanth, methyl cellulose, hydroxypropylmethyl-cellulose, sodium carboxymethylcellulose, and/or polyvinylpyrrolidone (PVP). Preferably, a non-cariogenic excipient is used.

Preferably a disintegrating agent may be added to a composition according to the invention. A suitable disintegrating agent includes a cross-linked polyvinyl pyrrolidone, agar, starch, carboxymethylcellulose, carragenan, carboxymethylcellulose calcium, croscarmellose sodium and/or carboxymethylstarch sodium.

A dyestuff and/or a pigment may optionally be added to a composition according to the invention to aid identification or to characterise a particular dosage type.

Optionally a binder is included in the composition according to the invention. A suitable binder includes a crystalline cellulose, sucrose, D-mannitol, dextrin, hydroxypropyl cellulose, hydroxypropyl methyl cellulose, and/or polyvinyl pyrrolidone.

Optionally a thickener or a viscosity builder is included in the composition according to the present invention. A suitable thickener or viscosity builder is a natural gum, cellulose derivative, and/or acrylic polymer.

Optionally a solubilizer may be included in the composition according to the invention. A suitable solubilizer is polyethylene glycol, polypropylene glycol, D-mannitol, benzyl benzoate, ethanol, trisaminomethane, cholesterol, triethanolamine, sodium carbonate and/or sodium citrate.

Optionally a buffer may be included in the composition according to the invention. A suitable buffer includes a phosphate, acetate, carbonate, and/or a citrate buffer solution.

Optionally a preservative may be included in the composition according to the invention. A suitable preservative includes a p-hydroxybenzoic ester, chlorobutanol, benzyl alcohol, phenethyl alcohol, dehydroacetic acid, and/or sorbic acid. Optionally an antioxidant may be included in the composition according to the invention. A suitable antioxidant is a sulfite and/or ascorbic acid. Where necessary, a further additive such as a preservative, antioxidant, colouring agent, sweetener and the like can be incorporated in the composition according to the invention.

The composition of the present invention may, if desired, be presented in a pack or dispenser device which may contain one or more unit dosage forms containing the active ingredient. The pack may, for example, comprise metal or plastic foil, such as a blister pack. The pack or dispenser device may be accompanied by instructions for administration.

A tablet, powder and/or granule may be manufactured by adding an excipient and one or more of a disintegrator, a binder and a lubricant to the extract according to the invention and compression-molding the mixture.

A composition according to the invention in the form of a quick dissolve tablet may be prepared, for example, by mixing a composition or extract according to the invention with an agent such as a sugar and/or a cellulose derivative, which promote dissolution or disintegration of the resultant tablet after oral administration, usually within 30 seconds.

A composition according to the invention in the form of a chewable tablet may be prepared by mixing the composition or extract according to the invention with one or more excipients designed to form a relatively soft, flavoured, tablet dosage form that is intended to be chewed rather than swallowed. Conventional tablet machinery and procedures, that is both direct compression and granulation, or slugging, before compression, can be utilized. Those individuals involved in pharmaceutical solid dosage form production are well versed in the processes and the machinery used as the chewable dosage form is a very common dosage form in the pharmaceutical industry.

A composition according to the invention in the form of a compressed tablet may be prepared by mixing the composition or extract according to the invention with one or more excipients intended to add binding qualities to disintegration qualities. The mixture is either directly compressed or granulated then compressed using methods and machinery well known to those in the industry. The resultant compressed tablet dosage units are then packaged according to market need, for example, as a unit dose, a roll, bulk bottle, or a blister pack.

### Brief Description of the Drawings

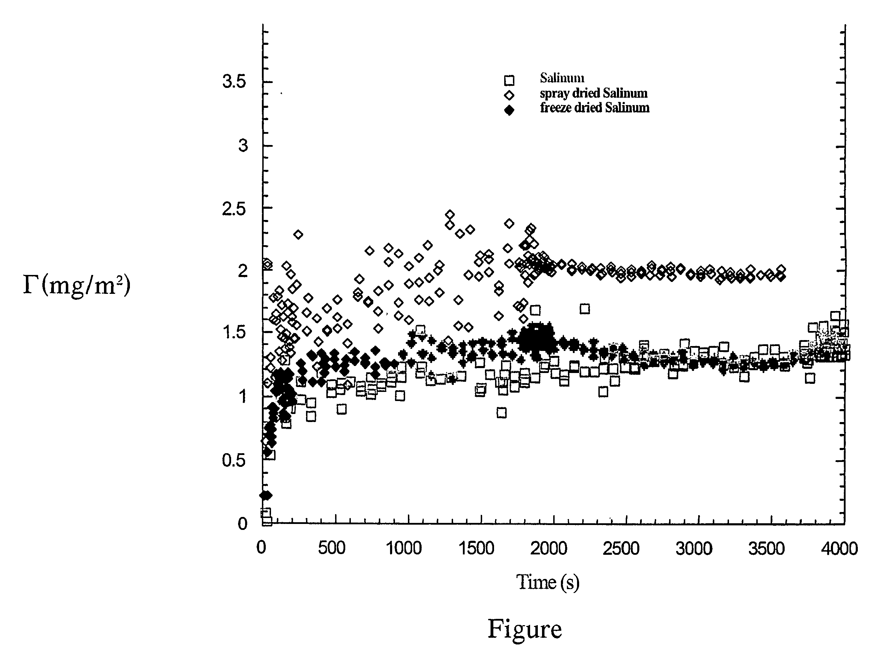

The invention is illustrated with reference to the Figure of the accompanying drawings which is not intended to limit the scope of the invention claimed and which shows the adsorption of redissolved linseed extract formulations (10%) on silica. Rinsing was started at 1800 seconds for the mixture.

### Materials and methods

### Materials

A liquid linseed extract formulation, made in accordance with the process described in US Patent No. 5,260,282, was used as starting material and was provided by Biosurface Pharma AB.

### Ellipsometry

Ideally film formation should be measured on a soft tissue such as the inside of a lip. There exists, however, no method that can do this with accuracy. Ellipsometry is very accurate in order to measure surface load, but requires an optically reflecting surface. Silica surfaces, used in the present experiments, have been shown to accumulate salivary material in a way similar to hydroxyapatite, which is the major inorganic component in tooth enamel.

Ellipsometry is an optical method to measure the changes in polarisation of light upon reflection at a surface (Azzam R M A, Bashara N M, "Ellipsometry and polarised light", North-Holland Amsterdam, 1977). The instrument used was a Rudolph thin film ellipsometer, type 436 (Rudolph Research, Fairfield, N. J.), equipped with a xenon lamp filtered to 4015 Å. To determine the ellipsometric angles, Δ and ψ for the bare substrate, the position of the intensity minimum was established. From the changes in Δ and ψ, compared to the clean substrate, the thickness and refractive index of a thin film can be calculated according to McCrackin et al. (McCrackin F L, Passaglia E, Stromberg R R, Steinberg H L, J. Res. Nat. Bur. Stand. (1963); A67:363). The adsorbed amount was calculated according to Cuypers et al. (Cuypers P A, Corsel J W, Janssen M P, Kop J M M, Hermens W T, Hemker H C, J. Biol. Chem. (1983); 258:2426) using values for the ratio between molar weight and molar refractivity and for the partial specific volume of 4.1 g/ml and 0.75 ml/g, respectively. Stock solutions were added to milli-Q water, unless otherwise stated, to give 5 ml solution in the ellipsometer cuvette with a protein concentration of 10%. Hydrophilic silica surfaces with an oxide layer of 300 to 350 Å, obtained by thermal oxidation of silicon test slides (p-type, boron doped, resistivity 1-20 Ωcm), were used as substrates.

The hydrophilic silica surfaces were cleaned according to the following procedure: The surfaces were immersed for 5 min at 80°C first in NH₄:H₂O₂:H₂O (1:1:5) (v/v/v) and then in HCl:H₂O₂:H₂O (1:1:5) (v/v/v) with subsequent rinsing in water and after the last step rinsing in ethanol. The cleaned surfaces were stored in ethanol. Immediately prior to use the surface was rinsed in ethanol and water and after drying in the flow of dry nitrogen, plasma cleaned in low pressure residual air, using a radio frequency glow discharge unit (Harrick PDC 3XG, Harrick Scientific Corp., Ossining, New York). As was obvious from their water wettability the surfaces were hydrophilic.

### Spray-drying

Linseed extract was spray dried in a conventional spray-dryer. The dimensions of the drying chamber are 0.5 x 0.15 m². The spray dryer operates co-currently and has a spray-nozzle with an orifice 1 mm in diameter. Inlet gas temperature was 180°C. Outlet gas temperature was kept at 80°C. Liquid feed to the dryer was 5 ml/min. The flow of drying air was 0.8 m³/min. Powder was collected in a cyclone at the outlet. Powders were stored at room temperature in closed containers within a desiccator.

### Freeze-drying

Freeze-drying was performed in a laboratory freeze-drier Lyovac GT 2 (Steris GmbH, Hurth, Germany). The samples were frozen separately at -80 °C and transferred to the freeze-drier in frozen state. Drying was performed at 0.1 mbar for 70 hours.

### EXAMPLES

### Test of spray dried and freeze dried linseed extract powders:

Adsorption properties of the aqueous re-dissolved formulations were measured by ellipsometry (as shown in the Figure) and compared to the same characteristics of a linseed extract prepared according to the examples of US 5,260,282.

Therefore, it can be seen that the adsorption behaviour of the linseed extract was affected slightly by freeze drying, whereas the spray-dried product gave a significantly higher adsorbed amount. The adsorption effects observed on the linseed extract of the present invention by drying are quite unexpected. It appears that the protein fraction in linseed extract plays a significant role.

Furthermore, the film forming properties of the spray-dried product were significantly better than both untreated and freeze-dried linseed extracts, as indicated by higher adsorbed amounts on silica. This unexpected behaviour is likely due to changes in conformation and/or association of proteins and shows that spray drying gives significant advantages. A higher adsorption value is useful because it shows that the linseed extract will have a longer lasting lubricant effect.

### Tablet formulations of linseed extract powders:

An example of a tablet composition according to the present invention was prepared by mixing the ingredients presented in Table 1. The tablets are referred to as Salinum tablets. Salinum is the trade name applied to the linseed extract utilised in the present invention.

**TABLE 1**

| Example of composition of Salinum tablets | |
|---|---|
| Ingredient | Amount (percent w/w) |
| Salinum dry substance | 3.3 |
| Isomalt | 53.1 |
| Xylitol | 37.4 |
| malic acid | 4.7 |
| magnesium stearate | 1.5 |

Typical laboratory batch productions of Salinum tablets were started with dry Salinum powder and grinding it at low temperature in a mortar together with xylitol. This was followed by mixing with isomalt and malic acid in a turbula mixer for 10 minutes, and finally by addition of magnesium stearate including further 2 minutes of turbula mixing. The granulate was then transferred to an eccentric tablet press (Diaf TM-20).

Salinum protects and lubricates hard and soft surfaces of the oral cavity due to its composition of polysaccharides and proteins. The function of these components is to increase viscosity and provide film formation through surface activity, respectively. An important property of Salinum is its ability to form such films on different types of surfaces, which is important for its effectiveness. This also contributes to the comparatively long duration of residence of Salinum.

## Claims

1. Use of a water-soluble or water-dispersible linseed extract that has an adsorption of at least 1.2 mg/m² wherein the adsorption is measured by contacting an aqueous solution or dispersion of the extract with a silica substrate, rinsing the silica substrate and then measuring the adsorption by ellipsometry, in the manufacture of a solid medicament for the treatment of xerostomia by direct administration on the medicament in solid form.

2. Use according to claim 1 wherein the adsorption of the extract is from 1.3 to 5 mg/m², preferably from 1.4 to 4 mg/m², more preferably from 1.5 to 3 mg/m².

3. Use according to claim 1 or claim 2 wherein the extract has been spray dried or freeze dried.

4. Use according to any one of the preceding claims wherein the adsorption of the extract is from 1.75 to 2.5 mg/m², preferably the adsorption is about 2 mg/m².

5. Use according to any one of the preceding claims wherein in the adsorption measurement method, there is a contact time between when the aqueous solution or dispersion of the extract contacts the silica substrate and when the silica substrate is rinsed wherein the contact time is from 100 to 3000 seconds, preferably from 500 to 2500 seconds, more preferably from 1000 to 2000 seconds.

6. Use according to any preceding claim, wherein the medicament further comprises a pharmaceutically acceptable excipient.

7. Use according to claim 6, wherein the medicament comprises less than 10% water by weight, preferably less than 5% water by weight, more preferably less than 2% water by weight, most preferably less than 1 % water by weight.

8. Use according to any preceding claim, wherein the medicament is presented in the form of a tablet, capsule or a powder.

## Patentansprüche

1. Verwendung eines wasserlöslichen oder wasserdispergierbaren Leinsamenextrakts, der eine Adsorption von mindestens 1,2 mg/m² aufweist, wobei die Adsorption gemessen wird, indem eine wässerige Lösung oder Dispersion des Extrakts mit einem Silica-Substrat in Berührung gebracht wird, das Silica-Substrat gespült wird und dann die Adsorption mittels Ellipsometrie gemessen wird, bei der Herstellung eines festen Arzneimittels zur Behandlung von Xerostomie mittels direkter Verabreichung des Arzneimittels in fester Form.

2. Verwendung gemäß Anspruch 1, wobei die Adsorption des Extrakts zwischen 1,3 bis 5 mg/m² liegt, vorzugsweise zwischen 1,4 bis 4 mg/m², bevorzugter zwischen 1,5 bis 3 mg/m².

3. Verwendung gemäß Anspruch 1 oder Anspruch 2, wobei der Extrakt sprühgetrocknet oder gefriergetrocknet wurde.

4. Verwendung gemäß einem der vorhergehenden Ansprüche, wobei die Adsorption des Extrakts zwischen 1,75 bis 2,5 mg/m² liegt, vorzugsweise liegt die Adsorption bei ca. 2 mg/m².

5. Verwendung gemäß einem der vorhergehenden Ansprüche, wobei es bei der Messmethode der Adsorption eine Berührungszeit dazwischen gibt, wenn die wässerige Lösung oder Dispersion des Extrakts das Silica-Substrat berührt, und wenn das Silica-Substrat gespült wird, wobei die Berührungszeit zwischen 100 und 3000 Sekunden liegt, vorzugsweise zwischen 500 bis 2500 Sekunden, bevorzugter zwischen 1000 bis 2000 Sekunden.

6. Verwendung gemäß einem vorhergehenden Anspruch, wobei das Arzneimittel zusätzlich einen pharmazeutisch annehmbareren Trägerstoff umfasst.

7. Verwendung gemäß Anspruch 6, wobei das Arzneimittel weniger als 10 Gew.-% Wasser umfasst, vorzugsweise weniger als 5 Gew.-% Wasser, bevorzugter weniger als 2 Gew.-% Wasser, am bevorzugtesten 1 Gew.-% Wasser.

8. Verwendung gemäß einem vorhergehenden Anspruch, wobei das Arzneimittel als Tablette, Kapsel oder Pulver angeboten wird.

## Revendications

1. Utilisation d'un extrait de graine de lin soluble à l'eau ou dispersable dans l'eau possédant une absorption minimum d'1.2 mg/m², lorsque l'absorption est mesurée au contact d'une solution ou dispersion aqueuse de l'extrait avec un substrat de silice, puis en rinçant le substrat et en mesurant l'absorption par ellipsométrie, lors de la fabrication d'un médicament solide pour le traitement de la xérostomie par administration directe du médicament sous forme solide.

2. Utilisation selon revendication 1, où l'absorption de l'extrait va de 1.3 à 5mg/m², de préférence de 1.4 à 4mg/m², et au mieux de 1.5 à 3mg/m².

3. Utilisation selon revendication 1 ou revendication 2, où l'extrait a été séché par aspersion ou par congélation.

4. Utilisation selon une quelconque des revendications ci-dessus, où l'absorption de l'extrait va de 1.75 à 2.5mg/m², avec une préférence pour une absorption d'environ 2mg/m².

5. Utilisation selon une quelconque des revendications ci-dessus, où dans la méthode de mesure d'absorption, il se trouve un temps de contact entre le moment où la solution ou dispersion aqueuse de l'extrait entre en contact avec le substrat de silice et le moment où le substrat de silice est rincé, le temps de contact allant de 100 à 3000 secondes, de préférence de 500 à 2500 secondes, et au mieux de 1000 à 2000 secondes.

6. Utilisation selon une quelconque des revendications ci-dessus, où le médicament comporte aussi un excipient acceptable pharmaceutiquement.

7. Utilisation selon revendication 6, où le médicament comporte moins de 10% d'eau par poids, de préférence moins de 5% d'eau par poids, et au mieux moins de 2% d'eau par poids, l'idéal étant moins de 1 % d'eau par poids.

8. Utilisation selon une quelconque des revendications ci-dessus, où le médicament est présenté sous forme de comprimé, de capsule ou de poudre.
